# EUROPEAN PATENT SPECIFICATION

(11) **EP 1 884 511 B1**
(45) Date of publication and mention of the grant of the patent: **18.01.2012**
(21) Application number: 06741861.6
(22) Date of filing: 15.05.2006
(51) Int. Cl.: C07D 231/10, C07D 261/06, C07D 275/02, C07D 413/04, C07D 417/04, C07D 419/04, A01N 43/56, A01N 43/80, A01P 3/00, A01P 5/00, A01P 7/00

(54) **AROMATIC ETHER COMPOUNDS,PREPARATION AND USAGE THEREOF**
AROMATISCHE ETHERVERBINDUNGEN, DEREN HERSTELLUNG UND VERWENDUNG
COMPOSES ETHER AROMATIQUES, LEUR PREPARATION ET LEUR UTILISATION

(30) Priority: 26.05.2005 CN 200510046515
(43) Date of publication of application: 06.02.2008
(73) Proprietor: Shenyang Research Institute of Chemical Industry Co., Ltd., Shenyang Liaoning 110021 (CN); Sinochem Corporation, Beijing 100045 (CN)
(72) Inventor: LIU, Changling, Shenyang,Liaoning 110021 (CN); LI, Miao, Shenyang,Liaoning 110021 (CN); LI, Zhinian, Shenyang,Liaoning 110021 (CN); GENG, Liwen, Shenyang,Liaoning 110021 (CN); ZHANG, Hong, Shenyang,Liaoning 110021 (CN); ZHOU, Defeng, Shenyang,Liaoning 110021 (CN); CUI, Dongliang, Shenyang,Liaoning 110021 (CN); LUO, Yanmei, Shenyang,Liaoning 110021 (CN)
(74) Representative: Stuart, Ian Alexander
(86) International application number: PCT/CN2006/000971
(87) International publication number: WO 2006/125370

(56) References cited:
- WO-A1-99/33812
- CN-A- 1 030 749
- CN-A- 1 657 524
- CN-C- 1 046 275
- CN-C- 1 100 761
- DE-A1- 19 548 786
- US-A- 6 054 592

## Description

### FIELD OF THE INVENTION

The invention relates to fungicide or insecticide, specifically to an aryl ether compounds and their preparation and use thereof.

### BACKGROUND OF THE INVENTION

Methoxyacrylate compounds are natural products and known with biological active. Fungicide pyraclostrobin with broad spectrum was disclosed in US5869517, US6054592, CN1154692 and CN1308065. The structure of pyraclostrobin is as follows:

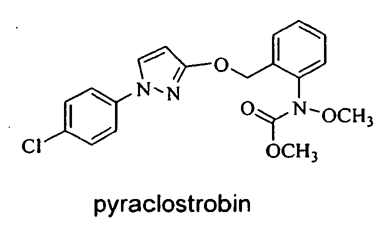

The following Compound with fungicidal activity was also disclosed in DE19548786:

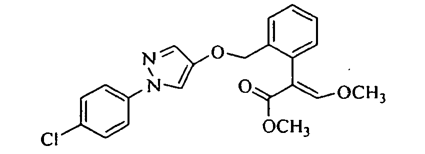

The following Compound with fungicidal activity is known in patent WO9933812:

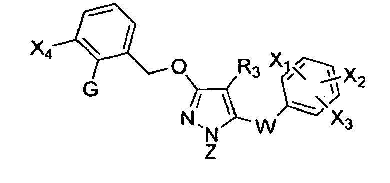

The following Compound with fungicidal activity is known in Nongyaoxue Xuebao, 2004, 6(1), 17-21:

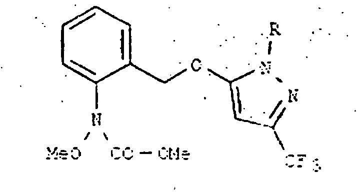

Before this invention, we applied CN1657524A and W02005080344A, which relates to fungicide or insecticide, especifically to substituted azole compounds having general formula, below: 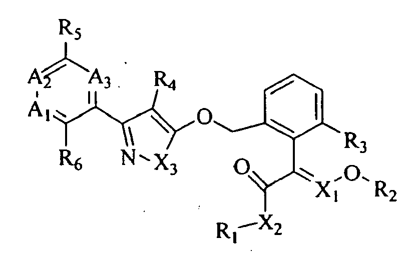 wherein: X₁ is selected from CH or N; X₂ is selected from O, S or NR₇; R₇ is selected from H or C₁-C₁₂alkyl; X₃ is selected from O, S or NR₈; R₈ is selected from H, C₁-C₁₂alkyl; C₁-C₁₂haloalkyl; C₁-C₁₂alkoxycarbonyl or C₁-C₁₂alkoxycarbonylC₁-C₁₂alkyl; R₁, R₂ is independently selected from H, C₁-C₁₂alkyl or C₁-C₁₂haloalkyl; A₁ is selected from N or CR₉; A₂ is selected from N or CR₁₀; A₃ is selected from N or CR₁₁; if A₁ is selected from N, A₂ is selected from CR₁₀, A₃ is selected from CR₁₁; if A₂ is selected from N, A₁ is selected from CR₉, A₃ is selected from CR₁₁; if A₃ is selected from N, A₁ is selected from CR₉, A₂ is selected from CR₁₀; R₃ is selected from H, halo, C₁-C₁₂alkyl, C₁-C₁₂haloalkyl or C₁-C₁₂alkoxy; R₄, R₅, R₆, R₉, R₁₀ and R₁₁ may be the same or different, selected from H, halo, NO₂, CN, CONH₂, CH₂CONH₂, CH₂CN, C₁-C₁₂alkyl, C₁-C₁₂haloalkyl, C₁-C₁₂alkoxy, C₁-C₁₂haloalkoxy, C₁-C₁₂alkylthio, C₁-C₁₂alkylsulfonyl, C₁-C₁₂alkylcarbonyl, C₁-C₁₂alkoxyC₁-C₁₂alkyl, C₁-C₁₂alkoxycarbonyl, C₁-C₁₂alkoxycarbonylC₁-C₁₂alkyl, C₁-C₁₂haloalkoxyC₁-C₁₂alkyl, or the group may be substituted by any other group consisting of aminoC₁-C₁₂alkyl, aroxyl, arylC₁-C₁₂alkoxy, aryl, heteroaryl, arylC₁-C₁₂alkyl, heteroarylC₁-C₁₂alkyl, heteroarylC₁-C₁₂alkoxy.

The compounds disclosed above patents (applications) were similar to this invention, but there are some obvious differences.

### SUMMARY OF THE INVENTION

The aim of the present invention is to provide the aryl ether compounds with biological activity against all sorts of plant disease and insects at very low dosage and the compounds can be applied in agriculture to control disease and insects in plant.

### Detailed description of the invention is as follows:

The present invention offered an.aryl ether compounds having general formula I:

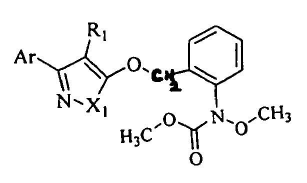

Wherein: X₁ is selected from O, S or NR₂;
R₁ is selected from H, halo, NO₂, CN, CONH₂, CH₂CONH₂, CH₂CN; C₁-C₁₂alkyl,
C₁-C₁₂haloalkyl, C₁-C₁₂alkoxy, C₁-C₁₂haloalkoxy, C₁-C₁₂alkylthio, C₁-C₁₂alkylsulfonyl,
C₁-C₁₂alkylcarbonyl, C₁-C₁₂alkoxyC₁-C₁₂alkyl, C₁-C₁₂alkoxycarbonyl,
C₁-C₁₂alkoxycarbonylC₁-C₁₂alkyl, C₁-C₁₂haloalkoxyC₁-C₁₂alkyl, substituted or unsubstituted aminoC₁-C₁₂alkyl;
R₂ is selected from H, C₁-C₁₂alkyl, C₁-C₁₂haloalkyl, C₁-C₁₂alkoxycarbonyl or C₁-C₁₂alkoxycarbonylC₁-C₁₂alkyl;
Ar is selected from substituted or unsubstituted aryl or heteroaryl, wherein the substitution group, if present, is selected from halo, CN, NO₂, C₁-C₆alkyl, C₁-C₆alkoxy, C₁-C₆alkylthio, C₁-C₆alkylsulfonyl, C₁-C₆alkoxycarbonyl, C₁-C₆haloalkoxy, substituted aryl or substituted aroxyl.

The preferred compounds of general formula I of this invention are:
X₁ is selected from O, S or NR₂;
R₁ is selected from H, halo, NO₂, CN, CONH₂, CH₂CONH₂, CH₂CN; C₁-C₆alkyl, C₁-C₆haloalkyl, C₁-C₆alkoxy, C₁-C₆haloalkoxy, C₁-C₆alkylthio, C₁-C₆alkylsulfonyl, C₁-C₆alkylcarbonyl, C₁-C₆alkoxyC₁-C₆alkyl, C₁-C₆alkoxycarbonyl. C₁-C₆alkoxycarbonylC₁-C₆alkyl, C₁-C₆haloalkoxyC₁-C₆alkyl, substituted or unsubstituted aminoC₁-C₆alkyl;
R₂ is selected from H, C₁-C₆alkyl, C₁-C₆haloalkyl, C₁-C₆alkoxycarbonyl or C₁-C₆alkoxycarbonylC₁-C₆alkyl;

Ar is selected from substituted or unsubstituted aryl or heteroaryl; the substitution group, if present, being selected from halo, CN, NO₂, C₁-C₆alkyl, C₁-C₆alkoxy, C₁-C₆alkylthio C₁-C₆alkylsulfonyl, C₁-C₆alkoxycarbonyl, C₁-C₆haloalkoxy, substituted phenyl or substituted phenoxy.

Further more, the preferred compounds of general formula I of this invention are:
X₁ is selected from O or NR₂;
R₁ is selected from H, halo, NO₂, CN, CONH₂, CH₂CONH₂, CH₂CN; C₁-C₆alkyl, C₁-C₆haloalkyl, C₁-C₆alkoxy, C₁-C₆haloalkoxy, C₁-C₆alkylthio, C₁-C₆alkylsulfonyl, C₁-C₆alkylcarbonyl. C₁-C₆alkoxyC₁-C₆alkyl, C₁-C₆alkoxycarbonyl, C₁-C₆alkoxycarbonylC₁-C₆alkyl, C₁-C₆haloalkoxyC₁-C₆alkyl, substituted or unsubstituted aminoC₁-C₃alkyl;
R₂ is selected from H, C₁-C₆alkyl, C₁-C₆haloalkyl, C₁-C₃alkoxycarbonyl or C₁-C₆alkoxycarbonylC₁-C₃alkyl;
Ar is selected from substituted or unsubstituted phenyl, pyridine, furan, thiophen or thiazol; the substitution group may be selected from halo, CN; NO₂, C₁-C₆alkyl, C₁-C₆alkoxy, C₁-C₆alkylthio, C₁-C₆alkylsulfonyl, C₁-C₆haloalkoxy, substituted phenyl or substituted phenoxy.

Even more preferred compounds of formula I of this invention are:
X₁ is selected from O or NR₂;
R₁ is selected from H, Cl, Br, F, NO₂, CH₂CN or C₁-C₆alkyl;
R₂ is selected from H, C₁-C₃alkyl, C₁-C₃haloalkyl, C₁-C₃alkoxycarbonyl or C₁-C₃alkoxycarbonylC₁-C₃alkyl;

Ar is selected from substituted or unsubstituted phenyl, pyridine, furan, thiophen or thiazol; the substitution group may be selected from halo, CN, NO₂, C₁-C₆alkyl, C₁-C₆alkoxy, C₁-C₆alkylthio, C₁-C₆haloalkoxy, chloro phenyl or chloro phenoxy.

Most preferred compounds of formula I of this invention are:
X₁ is selected from O or NR₂;
R₁ is selected from H or methyl;
R₂ is selected from H, methyl or isopropyl; Ar is selected from substituted or unsubstituted phenyl, pyridine, furan or thiophen; the substitution group may be selected from halo, C₁-C₃alkyl, C₁-C₃alkoxy, C₁-C₃alkylthio or C₁-C₃haloalkoxy.

The following is the meaning of terms in the general formula I:
Amino may be substituted by 1-2 groups, selected from C₁-C₃alkyl, C₁-C₃alkoxy, haloC₁-C₃alkyl, haloC₁-C₃alkoxy, halo, NO₂ or CN.
Phenyl, benzyl, phenoxy, benzyloxy may be substituted by 1-5 groups, selected from alkyl, alkoxy, haloalkyl, haloalkoxy, halo, NO₂ or CN.

Halogen or halo is fluorine, chlorine, bromine or iodine.

The alkyl is to be understood as meaning straight-chain or branched alkyl, such as methyl, ethyl, propyl, isopropyl or tert-butyl.

The haloalkyl refers to straight or branched chain alkyl, in which hydrogen atom may be all or partly substituted with halogen, such as chloromethyl, dichloromethyl, trichloromethyl, fluoromethyl, difluoromethyl, trifluoromethyl.

The alkoxy refers to straight or branched chain alkyl, which is linked to the structure by oxygen atom.

The haloalkoxy refers to straight or branched chain alkoxy, in which hydrogen atom may be all or partly substituted with halogen, such as chloromethoxy, dichloromethoxy, trichloromethoxy, fluoromethoxy, difluoromethoxy, trifluoromethoxy, chlorofluoromethoxy or trifluoroethoxy.

The alkenyl refers to a straight or branched, having double bonds at any position such as vinyl or allyl. Substituted alkenyl includes arylvinyl which is substituted at any position with any group.

The alkynyl refers to a straight or branched, having triple bonds at any position. Such as ethynyl, propynyl. Substitued alkynyl includes arylethynyl which is substituted at any position with any group.

The aryl and aryl in arylalkyl, arylalkenyl, arylalkynyl, aryloxy and aryloxyalkyl include phenyl or naphthyl.

The hetero aryl in this invention refers to five member ring or six member ring containing one or more N, O, S hetero atoms. Such as pyridine, furan, pyrimidine, pyrazine, pyridazine, triazine, quinoline or benzofuran.

The present invention is explained by the following compounds in table 1, but without being restricted thereby.

**Table 1**

| 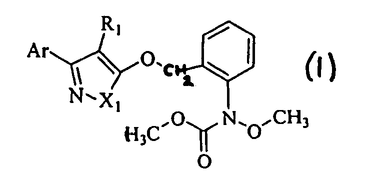 | | | |
|---|---|---|---|
| number | X₁ | R₁ | Ar |
| 1 | NCH₃ | H | C₆H₅ |
| 2 | NCH₃ | H | 4-Cl-C₆H₄ |
| 3 | NCH₃ | H | 4-F-C₆H₄ |
| 4 | NCH₃ | H | 4-NO₂-C₆H₄ |
| 5 | NCH₃ | H | 4-CF₃-C₆H₄ |
| 6 | NCH₃ | H | 4-CN-C₆H₄ |
| 7 | NCH₃ | H | 4-CH₃CO₂-C₆H₄ |
| 8 | NCH₃ | H | 4-CH₃S-C₆H₄ |
| 9 | NCH₃ | H | 4-CF₃O-C₆H₄ |
| 10 | NCH₃ | H | 2,4-2Cl-C₆H₃ |
| 11 | NCH(CH₃)₂ | H | 4-Cl-C₆H₄ |
| 12 | NCH₃ | H | 4-CH₃O-C₆H₄ |
| 13 | NCH₃ | H | 2-Cl-4-F-C₆H₃ |
| 14 | NCH₃ | H | 3-Cl-C₆H₄ |
| 15 | NCH₃ | H | 4-Br-C₆H₄ |
| 16 | NCH₃ | H | 4-CH₃-C₆H₄ |
| 17 | NCH₃ | H | 4-C₂H₅-C₆H₄ |
| 18 | NCH₃ | H | 4-CF₃CH₂O-C₆H₄ |
| 19 | NCH₃ | H | 4-PhO-C₆H₄ |
| 20 | NCH₃ | H | 2-Cl-C₆H₄ |
| 21 | NCH₃ | H | 6-CI-Pyridin-3-yl |
| 22 | NCH₃ | H | 6-F-Pyridin-3-yl |
| 23 | NCH₃ | H | 6-CF₃O-Pyridin-3-yl |
| 24 | NCH₃ | H | 6-CF₃-Pyridin-3-yl |
| 25 | NCH₃ | H | 6-CH₃O-Pyridin-3-yl |
| 26 | NCH₃ | H | 6-CF₃CH₂O-Pyridin-3-yl |
| 27 | NCH₃ | H | 4-(4-Cl-Ph)-C₆H₄ |
| 28 | NCH₃ | H | Thiophen-2-yl |
| 29 | NCH₃ | H | 5-Cl-Thiophen-2-yl |
| 30 | NCH₃ | H | Thiazol-2-yl |
| 31 | NCH₃ | H | Furan-2-yl |
| 32 | NCH₃ | Cl | C₆H₅ |
| 33 | NCH₃ | Cl | 4-Cl-C₆H₄ |
| 34 | NCH₃ | Cl | 4-F-C₆H₄ |
| 35 | NCH₃ | Cl | 4-NO₂-C₆H₄ |
| 36 | NCH₃ | Cl | 4-CF₃-C₆H₄ |
| 37 | NCH₃ | Cl | 4-CN-C₆H₄ |
| 38 | NCH₃ | Cl | 4-CH₃CO₂-C₆H₄ |
| 39 | NCH₃ | Cl | 4-CH₃S-C₆H₄ |
| 40 | NCH₃ | Cl | 4-CF₃O-C₆H₄ |
| 41 | NCH₃ | Cl | 2,4-2CI-C₆H₃ |
| 42 | NCH(CH₃)₂ | Cl | 4-Cl-C₆H₄ |
| 43 | NCH₃ | Cl | 4-CH₃O-C₆H₄ |
| 44 | NCH₃ | Cl | 2-Cl-4-F-C₆H₃ |
| 45 | NCH₃ | Cl | 3-Cl-C₆H₄ |
| 46 | NCH₃ | Cl | 4-Br-C₆H₄ |
| 47 | NCH₃ | Cl | 4-CH₃-C₆H₄ |
| 48 | NCH₃ | Cl | 4-C₂H₅-C₆H₄ |
| 49 | NCH₃ | Cl | 4-CF₃CH₂O-C₆H₄ |
| 50 | NCH₃ | Cl | 4-PhO-C₆H₄ |
| 51 | NCH₃ | Cl | 2-Cl-C₆H₄ |
| 52 | NCH₃ | Cl | 6-Cl-Pyridin-3-yl |
| 53 | NCH₃ | Cl | 6-F-Pyridin-3-yl |
| 54 | NCH₃ | Cl | 6-CF₃O-Pyridin-3-yl |
| 55 | NCH₃ | Cl | 6-CF₃-Pyridin-3-yl |
| 56 | NCH₃ | Cl | 6-CH₃O-Pyridin-3-yl |
| 57 | NCH₃ | Cl | 6-CF₃CH₂O-Pyridin-3-yl |
| 58 | NCH₃ | Cl | 4-(4-Cl-Ph)-C₆H₄ |
| 59 | NCH₃ | Cl | Thiophen-2-yl |
| 60 | NCH₃ | Cl | 5-Cl-Thiophen-2-yl |
| 61 | NCH₃ | Cl | Thiazol-2-yl |
| 62 | NCH₃ | Cl | Furan-2-yl |
| 63 | NCH₃ | CH₃ | C₆H₅ |
| 64 | NCH₃ | CH₃ | 4-Cl-C₆H₄ |
| 65 | NCH₃ | CH₃ | 4-F-C₆H₄ |
| 66 | NCH₃ | CH₃ | 4-NO₂-C₆H₄ |
| 67 | NCH3 | CH₃ | 4-CF₃-C₆H₄ |
| 68 | NCH₃ | CH₃ | 4-CN-C₆H₄ |
| 69 | NCH₃ | CH₃ | 4-CH₃CO₂-C₆H₄ |
| 70 | NCH₃ | CH₃ | 4-CH₃S-C₆H₄ |
| 71 | NCH₃ | CH₃ | 4-CF₃O-C₆H₄ |
| 72 | NCH₃ | CH₃ | 2,4-2Cl-C₆H₃ |
| 73 | NCH(CH₃)₂ | CH₃ | 4-Cl-C₆H₄ |
| 74 | NCH₃ | CH₃ | 4-CH₃O-C₆H₄ |
| 75 | NCH₃ | CH₃ | 2-Cl-4-F-C₆H₃ |
| 76 | NCH₃ | CH₃ | 3-Cl-C₆H₄ |
| 77 | NCH₃ | CH₃ | 4-Br-C₆H₄ |
| 78 | NCH₃ | CH₃ | 4-CH₃-C₆H₄ |
| 79 | NCH₃ | CH₃ | 4-C₂H₅-C₆H₄ |
| 80 | NCH₃ | CH₃ | 4-CF₃CH₂O-C₆H₄ |
| 81 | NCH₃ | CH₃ | 4-PhO-C₆H₄ |
| 82 | NCH₃ | CH₃ | 2-Cl-C₆H₄ |
| 83 | NCH₃ | CH₃ | 6-Cl-Pyridin-3-yl |
| 84 | NCH₃ | CH₃ | 6-F-Pyridin-3-yl |
| 85 | NCH₃ | CH₃ | 6-CF₃O-Pyridin-3-yl |
| 86 | NCH₃ | CH₃ | 6-CF₃-Pyridin-3-yl |
| 87 | NCH₃ | CH₃ | 6-CH₃O-Pyridin-3-yl |
| 88 | NCH₃ | CH₃ | 6-CF₃CH₂O-Pyridin-3-yl |
| 89 | NCH₃ | CH₃ | 4-(4-Cl-Ph)-C₆H₄ |
| 90 | NCH₃ | CH₃ | Thiophen-2-yl |
| 91 | NCH₃ | CH₃ | 5-Cl-Thiophen-2-yl |
| 92 | NCH₃ | CH₃ | Thiazol-2-yl |
| 93 | NCH₃ | CH₃ | Furan-2-yl |
| 94 | O | H | C₆H₅ |
| 95 | O | H | 4-Cl-C₆H₄ |
| 96 | O | H | 4-F-C₆H₄ |
| 97 | O | H | 4-NO₂-C₆H₄ |
| 98 | O | H | 4-CF₃-C₆H₄ |
| 99 | O | H | 4-CN-C₆H₄ |
| 100 | O | H | 4-CH₃CO₂-C₆H₄ |
| 101 | O | H | 4-CH₃S-C₆H₄ |
| 102 | O | H | 4-CF₃O-C₆H₄ |
| 103 | O | H | 2,4-2Cl-C₆H₃ |
| 104 | O | H | 4-CH₃O-C₆H₄ |
| 105 | O | H | 2-Cl-4-F-C₆H₃ |
| 106 | O | H | 3-Cl-C₆H₄ |
| 107 | O | H | 4-CH₃-C₆H₄ |
| 108 | O | H | 4-Br-C₆H₄ |
| 109 | O | H | 4-C₂H₅-C₆H₄ |
| 110 | O | H | 4-CF₃CH₂O-C₆H₄ |
| 111 | O | H | 4-PhO-C₆H₄ |
| 112 | O | H | 2-Cl-C₆H₄ |
| 113 | O | H | 6-Cl-Pyridin-3-yl |
| 114 | O | H | 6-F-Pyridin-3-yl |
| 115 | O | H | 6-CF₃O-Pyridin-3-yl |
| 116 | O | H | 6-CF₃-Pyridin-3-yl |
| 117 | O | H | 6-CH₃O-Pyridin-3-yl |
| 118 | O | H | 6-CF₃CH₂O-Pyridin-3-yl |
| 119 | O | H | 4-(4-Cl-Ph)-C₆H₄ |
| 120 | O | H | Thiophen-2-yl |
| 121 | O | H | 5-Cl-Thiophen-2-yl |
| 122 | O | H | Thiazol-2-yl |
| 123 | O | H | Furan-2-yl |
| 124 | O | Cl | C₆H₅ |
| 125 | O | Cl | 4-Cl-C₆H₄ |
| 126 | O | Cl | 4-F-C₆H₄ |
| 127 | O | Cl | 4-NO₂-C₆H₄ |
| 128 | O | Cl | 4-CF₃-C₆H₄ |
| 129 | O | Cl | 4-CN-C₆H₄ |
| 130 | O | Cl | 4-CH₃CO₂-C₆H₄ |
| 131 | O | Cl | 4-CH₃S-C₆H₄ |
| 132 | O | Cl | 4-CF₃O-C₆H₄ |
| 133 | O | Cl | 2,4-2Cl-C₆H₃ |
| 134 | O | Cl | 4-CH₃O-C₆H₄ |
| 135 | O | Cl | 2-Cl-4-F-C₆H₃ |
| 136 | O | Cl | 3-Cl-C₆H₄ |
| 137 | O | Cl | 4-CH₃-C₆H₄ |
| 138 | O | Cl | 4-Br-C₆H₄ |
| 139 | O | Cl | 4-C₂H₅-C₆H₄ |
| 140 | O | Cl | 4-CF₃CH₂O-C₆H₄ |
| 141 | O | Cl | 4-PhO-C₆H₄ |
| 142 | O | Cl | 2-Cl-C₆H₄ |
| 143 | O | Cl | 6-Cl-Pyridin-3-yl |
| 144 | O | Cl | 6-F-Pyridin-3-yl |
| 145 | O | Cl | 6-CF₃O-Pyridin-3-yl |
| 146 | O | Cl | 6-CF₃-Pyridin-3-yl |
| 147 | O | Cl | 6-CH₃O-Pyridin-3-yl |
| 148 | O | Cl | 6-CF₃CH₂O-Pyridin-3-yl |
| 149 | O | Cl | 4-(4-Cl-Ph)-C₆H₄ |
| 150 | O | Cl | Thiophen-2-yl |
| 151 | O | Cl | 5-Cl-Thiophen-2-yl |
| 152 | O | Cl | Thiazol-2-yl |
| 153 | O | Cl | Furan-2-yl |
| 154 | O | CH₃ | C₆H₅ |
| 155 | O | CH₃ | 4-Cl-C₆H₄ |
| 156 | O | CH₃ | 4-F-C₆H₄ |
| 157 | O | CH₃ | 4-NO₂-C₆H₄ |
| 158 | O | CH₃ | 4-CF₃-C₆H₄ |
| 159 | O | CH₃ | 4-CN-C₆H₄ |
| 160 | O | CH₃ | 4-CH₃CO₂-C₆H₄ |
| 161 | O | CH₃ | 4-CH₃S-C₆H₄ |
| 162 | O | CH₃ | 4-CF₃O-C₆H₄ |
| 163 | O | CH₃ | 2,4-2Cl-C₆H₃ |
| 164 | O | CH₃ | 4-CH₃O-C₆H₄ |
| 165 | O | CH₃ | 2-Cl-4-F-C₆H₃ |
| 166 | O | CH₃ | 3-Cl-C₆H₄ |
| 167 | O | CH₃ | 4-CH₃-C₆H₄ |
| 168 | O | CH₃ | 4-Br-C₆H₄ |
| 169 | O | CH₃ | 4-C₂H₅-C₆H₄ |
| 170 | O | CH₃ | 4-CF₃CH₂O-C₆H₄ |
| 171 | O | CH₃ | 4-PhO-C₆H₄ |
| 172 | O | CH₃ | 2-Cl-C₆H₄ |
| 173 | O | CH₃ | 6-Cl-Pyridin-3-yl |
| 174 | O | CH₃ | 6-F-Pyridin-3-yl |
| 175 | O | CH₃ | 6-CF₃O-Pyridin-3-yl |
| 176 | O | CH₃ | 6-CF₃-Pyridin-3-yl |
| 177 | O | CH₃ | 6-CH₃O-Pyridin-3-yl |
| 178 | O | CH₃ | 6-CF₃CH₂O-Pyridin-3-yl |
| 179 | O | CH₃ | 4-(4-Cl-Ph)-C₆H₄ |
| 180 | O | CH₃ | Thiophen-2-yl |
| 181 | O | CH₃ | 5-Cl-Thiophen-2-yl |
| 182 | O | CH₃ | Thiazol-2-yl |
| 183 | O | CH₃ | Furan-2-yl |
| 184 | NCH₃ | H | 2-CH₃O-C₆H₄ |
| 185 | NCH₃ | H | 2,5-2CH₃-Thiophen-3-yl |
| 186 | NCH₃ | H | 2,4-2CH₃-C₆H₃ |
| 187 | NCH₃ | H | 3,4-2CH₃-C₆H₃ |
| 188 | NCH₃ | H | 2,5-2CH₃-C₆H₃ |
| 189 | NCH₃ | H | 2,6-2CH₃-C₆H₃ |
| 190 | NCH₃ | CH₃ | 2,4-2CH₃-C₆H₃ |
| 191 | NCH₃ | CH₃ | 3,4-2CH₃-C₆H₃ |
| 192 | NCH₃ | CH₃ | 2,5-2CH₃-C₆H₃ |
| 193 | NCH₃ | CH₃ | 2,6-2CH₃-C₆H₃ |

The present invention also includes preparation of the compounds having formula I.

Compounds having general formula I can be prepared by reaction of azole compounds containing hydroxy group having general formula III with halomethylbenzene having general formula IV in the present of base:

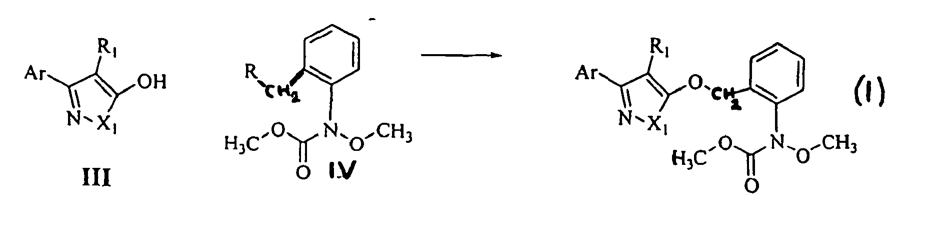

Wherein: R is leaving group, such as Cl or Br, other groups are as defined above.

The proper solvent mentioned above may be selected from tetrahydrofuran, acetonitrile, toluene, xylene, benzene, DMF, DMSO, acetone or butanone and so on.

The proper base mentioned above may be selected from potassium hydroxide, sodium hydroxide, sodium carbonate, potassium carbonate, sodium bicarbonate, triethylamine, pyridine, sodium methoxide, sodium ethoxide, sodium hydride, potassium or sodium tert-butoxide and so on.

The proper temperature mentioned above is from room temperature to boiling point of solvent, normal temperature is from 20 to 100°C.

The reaction may be finished after 30 minutes - 20 hours, 1-10 hours in general.

Intermediate of the general formula III can be prepared by reaction of intermediate of the general formula II with (substituted) hydrazine or hydroxylamine according to known methods. Intermediate of the general formula II can be bought or prepared according to known methods, refer to US Pat. No. 3781438, CN1257490, WO 9615115 and so on.

The intermediates having general formula IV can be prepared according to known methods, refer to US Pat. No. 4723034 and US Pat. No. 5554578 and so on.

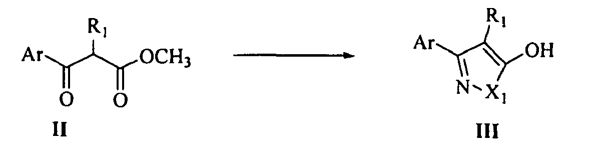

The compounds of the present invention have wide spectrum fungicidal activity, and may be used to control diseases in all sorts of plants caused by oomycete, basidiomycete, ascomycete pathogens and deuteromycete, and it may also provide good control efficacy at very low dosage because of the high activity. These compounds have penetration activity and can be used as soil and foliar fungicides. They can provide satisfactory control of cucumber downy mildew, cucumber grey mold, cucumber anthracnose, cucumber powdery mildew, tomato early blight, tomato late blight, phytophthora blight of pepper, grape downy mildew, grape white rot, apple ring rot, apple alternaria leaf spot, rice sheath blight, rice blast, wheat leaf rust, wheat leaf blotch, wheat powdery mildew, rape sclerotiniose and corn southern leaf blight.

Some of the compounds of the present invention have very good insecticidal and acaricidal activity, and may be used to control insects and mites.

The present invention also provides a composition of insecticides and fungicides. The active ingredients of the compositions are the compounds having general formula I with a carrier acceptable in agriculture, wherein the active ingredients being present in a total amount of 0.1 to 99% by weight.

The present invention, further more provides preparation methods of the said composition thereof. The compounds of general formula I and their carrier are mixed. The said composition may be a single component compound or mixture of compounds with several components of the invention.

The carrier should be easy to apply to the sites to be treated, after it is mixed with active component. For example, the sites could be plant, seed or soil or places convenient for store, transport or operation. The carrier could be solid or liquid, including the liquid which usually turns from gas condition under pressure.

Suitable solid carrier(s) includes natural or synthetic clays or silicates, for example diatomaceous earths, talcs, magnesium aluminium silicates, aluminium silicates(kaoling), montmorillonites and micas; calcium carbonate; calcium sulphate; ammonium sulphate; synthetic silicon oxides and synthetic calcium silicates or aluminium silicates; elements such as carbon or sulphur; natural and synthetic resins such as coumarone resins, polyvinyl chloride, or styrene polymers or copolymers; solid polychlorophenols; bitumen; waxes, beeswax or paraffin for instance.

Suitable liquid carriers include water, alcohols such as isopropanol or alcohol; ketones such as acetone, methyl ethyl ketone, methyl isopropy ketone or cyclohexanone; ethers; aromatics such as benzene, toluene, xylene; petroleum fractions such as kerosene or mineral oils;chlorinated aliphatic hydrocarbons such as carbon tetrachloride, tetrachloride ethylene or trichloride ethylene. Mixtures of these different liquids generally are often suitable.

The compositions of insecticides and fungicides are often formulated and transported in a concentrated form which is subsequently diluted by the user before application. The presence of small amounts of surfactant facilitates this process of dilution. Thus preferably at least one carrier in a composition according to the invention is a surfactant. For example the composition may contain at least two carriers, at least one of which is a surfactant.

The surfactant may be an emulsifier, a dispersant or a wetting agent; it may be nonionic or ionic. Examples of suitable surfactant include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycol, sorbic alcohol, sucrose or pentaerythritol and condensations of these with ethylene oxide and/or propylene oxide; condensation products of fatty alcohol or alkyl phenols such as p-octylphenol or p-octylcresol, with ethylene-oxide and/or propylene oxide; sulphates or sulphonates of these condensation products; alkaline metal salts or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate.

Examples of compositions and formulations according to the invention are wettable powder, dustable powder, granule, aqueous solution, emulsifiable concentrate, emulsion, suspension concentrate, aerosol composition or fumigant. Wettable powder usually contains 25, 50 or 75% by weight of active ingredient and usually contain in addition to solid inert carrier, 3-10% weight of a dispersant and, where necessary, 0-10% weight of stabiliser(s) and/or other additives such as penetrants or stickers. Dustable powder are usually formulated as a dust concentrate having a similar composition to that of a wettable powder but a dispersant, and are diluted further with solid carrier to give a composition usually containing 0.5-10% weight of active ingredient. Granules are usually prepared to have a size between 10 and 100 BS mesh (1.676-0.152 mm), and may be manufactured by agglomeration or impregnation techniques. Generally, granules contain 0.5-75% w active ingredient and 0-10% weight of additives such as stabilisers, surfactants, slow release modifiers. The so-called "dry flowable powders" consist of relatively small granules having a relatively high concentration of active ingredient. Emulsifiable concentrates usually contain, in addition to a solvent and, when necessary, co-solvent, 1-50% weight /volume (w/v) active ingredient, 2-20% w/v emulsifiers and 0-20% w/v of other additives such as stabilisers, penetrants and corrosion inhibitors. Suspension concentrates are usually contain 10-75% weight active ingredient, 0.5-15% weight of dispersing agents,0.1-10% weight of other additives such as defoamers, corrosion inhibitors, stabilisers, penetrants or stickers.

Aqueous dispersant and emulsions, for example compositions obtained by diluting a wettable powder or a concentrate according to the invention with water, also lie within the scope of the invention. The said emulsions may be of the water-in-oil or of the oil-in-water type.

The composition to which one or more other fungicides are added has wider spectrum activity than single compound having general formula I. In addition, other fungicides may have synergistic effect on the fungicidal activity of the compound having general formula I. The compound having general formula I can also be used with other insecticides, or with another fungicide and other insecticides simultaneously.

### DESCRIPTION OF THE INVENTION IN DETAIL

The following examples are illustrative of the present invention.

### Preparation Example

### Example 1 the preparation of compound 2

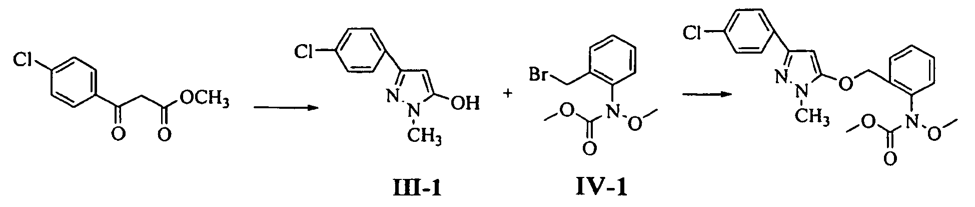

2.12g of methyl 3-(4-chlorophenyl)-3-oxopropanoate was dissolved in 10ml of methanol, the solution was heated to reflux. Slightly excessive methyl hydrazine was added to the solution dropwisely, 3 hours later, the reaction was traced by Thin-Layer Chromatography, after the reaction, the solution was condensed, cooled, crystal obtained, and filtrated. The residue was washed with methanol and dried, 1.5g crystal of 3-(4-chlorophenyl)-1-methyl-1*H*-pyrazol-5-ol (compound III-1) was obtained.

0.5g of the above crystal was dissolved in 5ml of DMF, and 0.17g of NaH was added to the solution. Then the solution was stirred for 30min. 0.72g of compound IV-1 was added, the mixture was stirred and heated to 40 °C. 3hr later, the reaction was traced by Thin-Layer Chromatography, after the reaction, the reaction mixture was poured into 50ml saturated brine, extracted with 60ml ethyl acetate 3 times. The combined organic extracts were dried, and concentrated. This was subjected to silica gel column chromatography; to obtain 0.56 g of oil (compound **2**).

¹HNMR spectrum (¹HNMR, 300Hz, internal standard: TMS, solvent CDCl₃) is as follows:
δppm 3.75(6H, s), 3.81 (3H, s), 5.20(2H, s), 5.86(1H, s), 7.35(2H, d), 7.44(3H, m), 7.54(1H, m), 7.69(2H, m).

### Example 2 the preparation of compound 21

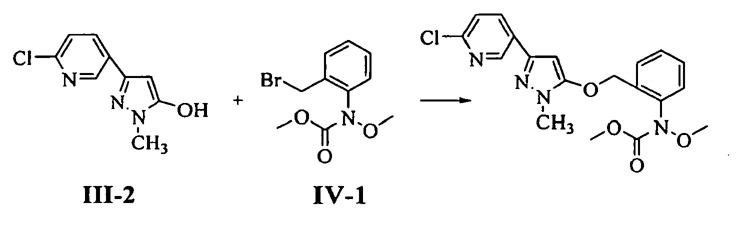

0.15g of NaH (60% pure) was charged in a flask, washed with petroleum ether, 5ml of DMF and 1.0g of compound III-2 (prepared according to CN1257490A). After stirred at room temperature for 2 min, 1.4g of compound IV-1 was added, the reaction temperature was rised to 60°C. 2hr later, the reaction was traced by Thin-Layer Chromatography, after the reaction, the reaction mixture was poured into 50ml saturated brine, extracted with 100ml ethyl acetate 3 times. The combined organic extracts were dried, and concentrated. The crude product was purified through silica gel column and 0.95g of oil (compound 21) was obtained.

¹HNMR spectrum (¹HNMR, 300Hz, internal standard: TMS, solvent CDCl₃) is as follows:
δppm 3.75(3H, s), 3.76(3H, s), 3.82(3H, s), 5.21(2H, s), 5.89(1H, s), 7.33(1H, d), 7.45(3H, m), 7.58 (1 H, d), 8.06(1H, d), 8.68(1H, s)o

### Example 3 the preparation of compound 89

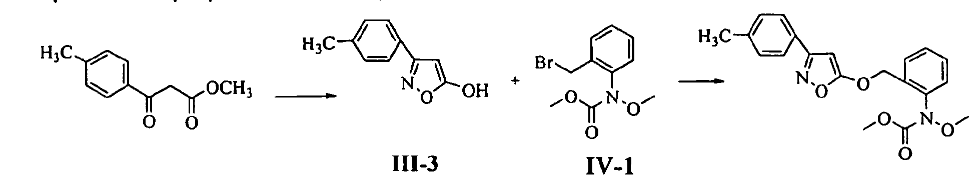

According to method of US Pat. No. 3781438, 2g of methyl 3-(4-methylphenyl)-3-oxopropanoate was dissolved in methanol, slightly excessive hydroxylamine hydrochloride and equivalent amount of sodium hydroxide were added, the mixture was heated to reflux. 3hr later, the reaction was traced by Thin-Layer Chromatography, after the reaction, water was added to the reaction mixture, extracted with ethyl acetate, The combined organic extracts were dried and concentrated to obtain 3-(4-methylphenyl)isoxazol-5-ol as solid(compound III-3).

1g of the above compound III-3 was dissolved in DMF, and 0.45g of NaH_was added to the solution, then the solution was stirred for 30min. 1.7g of compound IV-1 was added, the mixture was heated to 50°C and stirred for 6hr, the reaction was traced by Thin-Layer Chromatography, after the reaction, the reaction mixture was poured into 50ml saturated brine, extracted with 100ml ethyl acetate 3 times. The combined organic extracts were dried, and concentrated, the crude product was purified through silica gel column to obtain 1.5g of oil (compound 99).

### Example 4 the preparation of compound 121

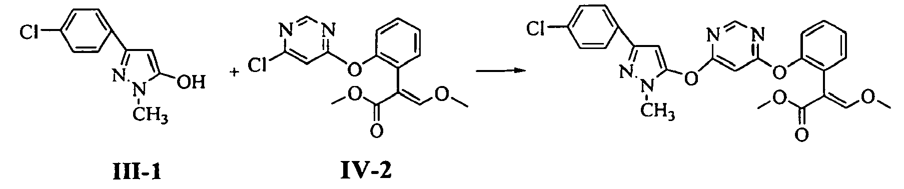

1.0g of compound III-1, 1.7g of compound IV-2 (prepared according to WO 9807707, WO 9208703), 2.1g of potassium carbonate were add in 15ml DMF, and the reaction mixture was heated to 70-80°C, 7hr later, the reaction was traced by Thin-Layer Chromatography, after the reaction turnover, the reaction mixture was poured into 100ml saturated brine, extracted with 100ml ethyl acetate for 3 times. The combined organic extracts were dried, and concentrated, the crude product was purified through silica gel column to obtain 1.1g of oil (compound 121).

Other compounds were prepared according the above examples.

Physical and chemical property and ¹HNMR spectrum (¹HNMR, 300Hz, internal standard:TMS, solvent CDCl₃) of some compounds having the general formula I in table 1 of this invention are as follows:
Compound 1: oil. δppm 3.75(6H, s), 3.81 (3H, s), 5.19(2H, s), 5.86(1H, s), 7.40(5H, m), 7.60(1 H, m), 7.68(2H, d).
Compound 8: m.p. 98-100°C. δppm 2.47(3H, s), 3.73(3H, s), 3.75(3H, s), 3.81 (3H, s), 5.18(2H, s), 5.83(1H, s), 7.24 (1H, m), 7.42(3H, m), 7.65(3H, m).
Compound 10: m.p. 89-91 °C. δppm3.75(6H, s), 3.79(3H, s), 5.20(2H, s), 6.07(1 H, s), 7.24(2H, d), 7.42(4H, m), 7.58(1H, m), 7.75(1H, d).
Compound 11: oil. δppm 1.45(6H, s), 3.60(3H, s), 3.66(3H, s), 4.94(2H, s), 7.22(3H, m), 7.30(3H, m), 7.47(3H, m).
Compound 12: oil. δppm 3.73(3H, s), 3.75(3H, s), 3.81(3H, s), 3.82(3H, s), 5.18(2H, s), 5.79(1H, s), 6.90(2H, m), 7.42(3H, m), 7.61(1H, m), 7.66(2H, m).
Compound 15: m.p. 90-92°C. δppm3.73(3H, s), 3.75(3H, s), 3.81 (3H, s), 5.18(2H, s), 5.83(1H, s), 7.43 (3H, m), 7.47(1H, m), 7.50(1H, m), 7.60(3H, m).
Compound 16: m.p. 93-95°C. δppm 2.36(3H, s), 3.74(3H, s), 3.76(3H, s), 3.82(3H, s), 5.19(2H, s), 5.83(1H, s), 7.19 (2H, d), 7.42(3H, m), 7.63(3H, d).
Compound 17: oil. δppm 1.27(3H, t), 1.89(3H, s), 2.70(2H, q), 3.62(3H, s), 3.78(6H, s) 5.32(2H, s), 7.22(2H, m), 7.28(2H, m), 7.40(3H, m), 7.70(1 H, m).
Compound 20: oil. δppm 3.57(6H, s), 3.79(6H, s), 5.26(2H, s), 5.73(1H, s), 7.37(6H, m), 7.50(1 H, m), 7.69(1 H, d).
Compound 26: m.p. 114-116°C. δppm 3.75(6H, s), 3.81(3H, s), 4.79(2H, t), 5.21(2H, s), 5.83(1H, s), 7.44(3H, m), 7.58(1H, d), 8.06(1H, dd), 8.44(1H, s).
Compound 28: oil. δppm 3.72(3H, s), 3.75(3H, s), 3.82(3H, s), 5.17(2H, s), 5.78(1H, s), 6.43(1 H, dd), 6.58(1H, m), 7.41 (4H, m), 7.59(1H, m).
Compound 29: oil. δppm 3.69(3H, s), 3.74(3H, s), 3.81(3H, s), 5.16(2H, s), 5.70(1H, s), 6.82(1H, m), 6.98(1H, m), 7.42(3H, m), 7.56(1H, m).
Compound 31: oil. δppm 3.73(3H, s), 3.76(3H, s), 3.82(3H, s), 5.18(2H, s), 5.78(1H, s); 7.01(1H, m), 7.20(1H, m), 7.24(1H, d), 7.42(3H, m), 7.60(1H, m).
Compound 63: m.p. 66-68°C. δppm 1.90(3H, s), 3.62(3H, s), 3.79(6H, s), 5.32(2H, s), 7.30(2H, d), 7.42(6H, m), 7.72(1H, d), 7.60(3H, m).
Compound 64: oil. δppm 1.68(3H, s), 3.60(3H, s), 3.79(6H, s), 5.30(2H, s), 7.25(2H, d), 7.42(5H, m), 7.72(1 H, d).
Compound 74: oil. δppm 1.88(3H, s), 3.61(3H, s), 3.78(3H, s), 3.79(3H, s), 3.86(3H, s), 5.32(2H, s), 6.99(2H, m), 7.22(2H, m), 7.40(3H, m), 7.69(1 H, m) .
Compound 78: oil. δppm 1.89(3H, s), 2.05(3H, s), 3.61(3H, s), 3.78(6H, s), 5.31(2H, s), 7.22(2H, d), 7.29 (1H, d), 7.38(3H, m), 7.71(1H, m).
Compound 107: oil. δppm 2.39(3H, s), 3.74(3H, s), 3.76(3H, s), 3.83(3H, s), 5.32(2H, s), 5.56(1H, s), 7.24 (2H, d), 7.42(3H, m), 7.63(3H, d).
Compound 184: oil. δppm 3.75(3H, s), 3.77(3H, s), 3.81(3H, s), 3.88(3H, s), 5.20(2H, s), 6.10(1H, s), 6.94(2H, m), 7.26(1H, m), 7.42(3H, m), 7.61 (1H, m), 7.87(1H, m).
Compound 185 oil. δppm 2.39(3H, s), 2.52(3H, s), 3.74(6H, s), 3.80(3H, s), 5.18(2H, s), 5.65(1H, s), 6.92(1H, s), 7.42(3H, m), 7.48(1H, m).

### Formulation example Base on 100% active ingredient (weight/weight%)

### Example 5 35% emulsion concentrate.

| | |
|---|---|
| Compound 2 (97.2%) | 35% |
| Phosphorous acid | 10% |
| Ethylenoxy aliphatic acid glycerin ester | 15% |
| Cyclohexanone | complement to 100% |

Phosphorous acid is dissolved in cyclohexanone, then the compound 2 and ethylenoxy aliphatic acid glycerin ester are added, the emulsifiable in transparent solution is obtained finally.

### Example 6 60% wettable powders

| | |
|---|---|
| Compound 64 (98.4%) | 60% |
| Sodium dodecylnaphthalenesulfate | 2% |
| Sodium lignosulfonate | 9% |
| Kaolin | complement to 100% |

Compound 64, sodium dodecylnaphthalenesulfate, sodium lignosulfonate and kaolin (all solid components) are well mixed and shattered until the particle size reaches the standard.

### Example 7 30% aqueous suspension

| | |
|---|---|
| Compound 121 (96.6%) | 30% |
| Sodium dodecylnaphthalenesulfate | 4 % |
| Hemicellulose | 2% |
| Epoxypropane | 8% |
| Water | complement to 100% |

The mixture of compound 121, 80% of the amount of water should being added and sodium dodecylnaphthalenesulfate are shattered in a mill (1mm ball). Other components are dissolved in the rest 20% water, and are added under stirring to obtain 30% aqueous suspension.

### Example 8 25% suspension emulsifier

| | |
|---|---|
| Compound 11 (96.2% pure) | 25% |
| Polyethylenoxyalkyl propyl ether | 2.5% |
| Dodecyl polyethylene phosphate ester | 4 % |
| Ethylenoxy aliphatic acid glycerin ester | 2% |
| Calcium dodecylbenzenesulfate | 1.5% |
| Cyclohexanone | 30% |
| Petroleum fractions | complement to 100% |

Compound 11 is dissolved in 80% of the amount of solvent (cyclohexanone and petroleum fractions) should being added, and then emulsifiers (dodecyl polyethylene phosphate ester, ethylenoxy aliphatic acid glycerin ester and calcium dodecylbenzenesulfate) and dispersant (2.5% weight of 2,3-epoxypropyl epoxy alkyl copolymer) are added, the mixture is stirred completely and shattered in a mill (1mm ball). And the rest 20% solvents are added.

### Biological testing

### Example 9 Determination of fungicidal activity

Determination of fungicidal activities against plant diseases of selected compounds were carried out by following procedure:

Protectant and curative activity of experimental compounds are tested with whole plants. Technical samples were dissolved in a bit acetone and diluted to required concentration with water containing 0.1 % Tween 80. For the protectant method, test solution was sprayed onto potted plant. Pathogen inoculation was carried out after 24 hours then plants were hold in growth chambers containing constant temperature and moisture for effect. When untreated plant was under desirable disease severity (after 1 week approximately), assessment were carried out by visual observation. Test plant inoculated with fungus for 4 days are sprayed with the solution of a compound and then cultured in green house for 3-4 days. The curative activity is assessed based on the control plant's index of disease.

Part of the test results:

At 500 mg/L, compound 16 showed 100% control against wheat powdery mildew(*BLumeria graminis*), compounds 1, 15, 26, showed more than 80%. At 500 mg/L, compounds 1, 8, 15, showed 100% control against cucumber downy mildew*(Pseudoperonospora cubenis),* compound 11 showed 90%.

At 500 mg/L, compound 21 showed 100% control against cucumber grey mold *(Botrytis cinerea*) and wheat powdery mildew.

At 500 mg/L, compounds 10, 20, 63, 78 showed 100% control against cucumber downy mildew and wheat powdery mildew.

At 25 mg/L, compounds 15, 20, 63, 78 showed 100% control against cucumber downy mildew. At 12.5 mg/L, they showed more than 95% control against wheat powdery mildew.

At 100 mg/L, compound 63 showed 100% control against cucumber anthracnose *(Colletotrichum Iagenarium*) and at 1.56 mg/L it still showed 100%.

Compound 63 showed excellent activity against rice blast (*Pyricularia oryzae*), tomato late blight (*Phytophthora infestans*), cucumber downy mildew (*Pseudoperonospora cubensis*), wheat powdery mildew (*Erysiphe graminis*), cucumber anthracnose (*Colletotrichum lagenarium*), cucumber powdery mildew (*Sphaerotheca fuliginea*), rice sheath blight (*Rhizoctonia solani*), and showed good activity on cucumber scab (*Cladosporium cucumerinum*), *apple* alternaria rot(valsa *mali*), rape sclerotinia stem rot (*Sclerotinia sclerotiorum*), and showed moderate activity against tomato cercospora leaf mold (*Fulvia fulva*), corn southern leaf blight (*Helminthosporium maydis*),wheat head blight (*Fusarium graminearum*).

The results of compound 63 against rice sheath blight and wheat powdery mildew are listed in table 2∼4.

**Table 2 the results of compound 63 against rice sheath blight**

| compound | 1 day protection activity (%) | | | | | | |
|---|---|---|---|---|---|---|---|
| | 100m | 50mg/ | 25mg/ | 12.5m | 6.25mg/ | 3.125mg/ | 1.5625 |
| **63** | 100 | 100 | 80 | 75 | 65 | 60 | 55 |
| triophanate-methyl | 75 | 55 | 20 | 0 | 0 | 0 | 0 |

**Table 3 the results of compound 63 against wheat powdery mildew**

| compound | 1 day protection activity (%) | | | | | | | |
|---|---|---|---|---|---|---|---|---|
| | 25mg /L | 12.5m g/L | 6.25mg /L | 3.125 mg/L | 1.56 mg/L | 0.78m g/L | 0.39m g/L | 0.19 mg/L |
| **63** | 100 | 100 | 100 | 100 | 100 | 95 | 75 | 55 |
| Kresoxim methyl | 100 | 100 | 98 | 70 | 40 | 15 | 0 | - |

**Table 4 the results of compound 63 against wheat powdery mildew**

| compound | 4 day curative activity (%) | | | | | |
|---|---|---|---|---|---|---|
| | 12 5mg/L | 6.25mg/ L | 3.125 mg/L | 1.56mg/ L | 0.78 mg/L | 0.39mg/L |
| **63** | 100 | 100 | 100 | 100 | 80 | 50 |
| tebuconazole | 85 | 70 | 45 | 15 | 0 | 0 |
| pyraclostrobin | 100 | 100 | 85 | 60 | 25 | 0 |
| Kresoxim methyl | 100 | 20 | 15 | 10 | 0 | 0 |

### Example 10 Determination of insecticidal and acaricidal activity

Determination of insecticidal and acaricidal activities of selected compounds were carried out by following procedure:

Technical samples were dissolved in mixed solvent (acetone:methanol=1:1), and diluted to required concentration with water containing 0.1 % of tween 80.

The second instar larvae of armyworm (*Leucania separata*), diamond backmoth (*Plutella xylostella*) and culex mosquitoes (*Culex pipiens pallens*), green peach aphids (*Myzus persicae*) and mite (*Tetranychus cinnabarinus*) were used in biological test. The method was employed either spraying by airbrush or immersing(larvae of culex mosquitoes). A test solution (0.5mL) was sprayed at the pressure of 10 psi (about 0.7 kg/cm² ). Percent mortality was determined after two days.

Part of test results:

At 600 mg/L, compound 15, 21, 63, 64 showed more than 85% control of armyworm, diamond backmoth, green peach aphids and culex mosquitoes.

At 200 mg/L, compound 15 showed 90% control of green peach aphids, and compound 63 showed 100% control of diamond backmoth.

## Claims

1. An aryl ether compound **characterized by** having formula (I): 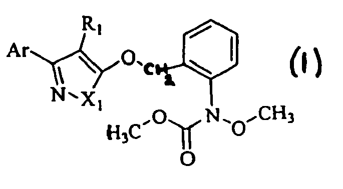 wherein: X₁ is selected from O, S or NR₂;
R₁ is selected from H, halo, NO₂, CN, CONH₂, CH₂CONH₂, CH₂CN; C₁-C₁₂alkyl, C₁-C₁₂haloalkyl, C₁-C₁₂alkoxy, C₁-C₁₂haloalkoxy, C₁-C₁₂alkylthio, C₁-C₁₂alkylsulfonyl, C₁-C₁₂alkylcarbonyl, C₁-C₁₂alkoxyC₁-C₁₂alkyl, C₁-C₁₂alkoxycarbonyl, C₁-C₁₂alkoxycarbonylC₁-C₁₂alkyl, C₁-C₁₂haloalkoxyC₁-Cₗ₂alkyl, or substituted or unsubstituted aminoC₁-C₁₂alkyl;
R₂ is selected from H, C₁-C₁₂alkyl, C₁-C₁₂haloalkyl, C₁-C₁₂alkoxycarbonyl or C₁-C₁₂alkoxycarbonylC₁-C₁₂alkyl;
Ar is selected from substituted or unsubstituted aryl or heteroaryl wherein the substitution group , if present is selected from halo, CN, NO₂, C₁-C₆alkyl, C₁-C₆alkoxy, C₁-C₆alkylthio, C₁-C₆alkylsulfonyl. C₁-C₆alkoxycarbonyl, C₁-C₆haloalkoxy, substituted aryl or substituted aroxyl.

2. The compound according to claim 1, wherein
R₁ is selected from H, halo, NO₂, CN, CONH₂, CH₂CONH₂, CH₂CN; C₁-C₆alkyl, C₁-C₆haloalkyl, C₁-C₆alkoxy, C₁-C₆haloalkoxy, C₁-C₆alkylthio, C₁-C₆alkylsulfonyl, C₁-C₆alkylcarbonyl, C₁-C₆alkoxyC₁-C₆alkyl, C₁-C₆alkoxycarbonyl, C₁-C₆alkoxycarbonylC₁-C₆alkyl. C₁-C₆haloalkoxyC₁-C₆alkyl,or substituted or unsubstituted aminoC₁-C₆alkyl;
R₂ is selected from H, C₁-C₆alkyl, C₁-C₆haloalkyl, C₁-C₆alkoxycarbonyl or C₁-C₆alkoxycarbonylC₁-C₆alkyl; and wherein if
Ar is selected from substituted aryl or heteroaryl; wherein the substitution group, if present is selected from halo, CN, NO₂, C₁-C₆alkyl, C₁-C₆alkoxy, C₁-C₆alkylthio, C₁-C₆alkylsulfonyl, C₁-C₆alkoxycarbonyl, C₁-C₆haloalkoxy, substituted phenyl or substituted phenoxy.

3. The compound according to claim 2, wherein X₁ is selected from O or NR₂;
R₁ is selected from H, halo, NO₂, CN, CONH₂, CH₂CONH₂, CH₂CN; C₁-C₆alkyl, C₁-C₆haloalkyl, C₁-C₆alkoxy, C₁-C₆haloalkoxy, C₁-C₆alkylthio, C₁-C₆alkylsulfonyl, C₁-C₆alkylcarbonyl, C₁-C₆alkoxyC₁-C₆alkyl, C₁-C₆alkoxycarbonyl, C₁-C₆alkoxycarbonylC₁-C₆alkyl, C₁-C₆haloalkoxyC₁-C₆alkyl, or substituted or unsubstituted aminoC₁-C₃alkyl;
R₂ is selected from H, C₁-C₆alkyl, C₁-C₆haloalkyl, C₁-C₃alkoxycarbonyl or C₁-C₆alkoxycarbonylC₁-C₃alkyl; and
Ar is selected from substituted or unsubstituted phenyl, pyridine, furan, thiophen or thiazol wherein the substitution groups, if present, is selected from halo, CN, NO₂, C₁-C₆alkyl, C₁-C₆alkoxy, C₁-C₆alkylthio, C₁-C₆alkylsulfonyl, C₁-C₆haloalkoxy, substituted phenyl or substituted phenoxy.

4. The compound according to the claim 3, wherein
R₁ is selected from H, Cl, Br, F, NO₂, CH₂CN or C₁-C₆alkyl;
R₂ is selected from H, C₁-C₃alkyl, C₁-C₃haloalkyl, C₁-C₃alkoxycarbonyl or C₁-C₃alkoxycarbonylC₁-C₃alkyl;
Ar is selected from substituted, or unsubstituted phenyl, pyridine, furan, thiophen or thiazol wherein the substitution group, if present, is selected from halo, CN, NO₂, C₁-C₆alkyl, C₁-C₆alkoxy, C₁-C₆alkylthio, C₁-C₆haloalkoxy, chloro phenyl or chloro phenoxy.

5. The compound according to claim 4, wherein
R₁ is selected from H or methyl;
R₂ is selected from H, methyl or isopropyl;
Ar is selected from substituted or unsubstituted phenyl, pyridine, furan or thiophen, wherein the substitution group, if present, is selected from halo, C₁-C₃alkyl, C₁-C₃alkoxy, C₁-C₃alkylthio or C₁-C₃haloalkoxy

6. A method of preparing a compound according to any of clams 1-5, **characterized in that** compounds having general formula(I) are prepared by reaction of azole compounds containing hydroxy group having general formula (III) with halomethylbenzene having general formula (IV) in the presence of base: 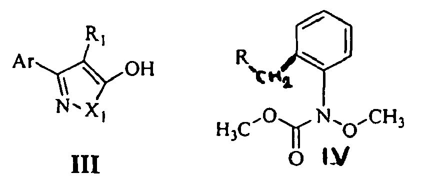 wherein: R is a leaving group.

7. A method according to claim 6 wherein R is Cl or Br.

8. Use of a compounds having general formula (I) according to claim 1 for controlling insects in plants.

9. Use of a compound having general formula (I) according to claim 1 for controlling fungi in plants.

10. A fungicidal and insecticidal composition containing a compound having general formula (I) as defined in claim 1 as an active ingredient, wherein the weight percentage of the active ingredient in the composition is from 0.1% to 99%.

## Patentansprüche

1. Aryletherverbindung, **dadurch gekennzeichnet, dass** sie die Formel (I) aufweist: 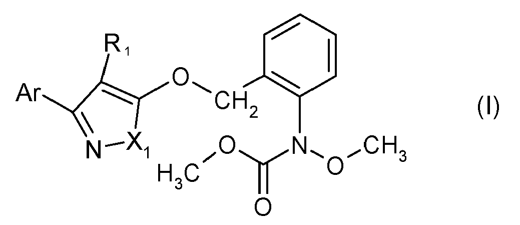 worin
X₁ aus O, S und NR₂ ausgewählt ist;
R₁ aus H, Halogen, NO₂, CN, CONH₂, CH₂CONH₂, CH₂CN, C₁-C₁₂-Alkyl, C₁-C₁₂-Halogenalkyl, C₁-C₁₂-Alkoxy, C₁-C₁₂-Halogenalkoxy, C₁-C₁₂-Alkylthio, C₁-C₁₂-Alkylsulfonyl, C₁-C₁₂-Alkylcarbonyl, C₁-C₁₂-Alkoxy-C₁-C₁₂-alkyl, C₁-C₁₂-Alkoxycarbonyl, C₁-C₁₂-Alkoxycarbonyl-C₁-C₁₂-alkyl, C₁-C₁₂-Halogenalkoxy-C₁-C₁₂-alkyl und substituiertem oder unsubstituiertem Amino-C₁-C₁₂-alkyl ausgewählt ist;
R₂ aus H, C₁-C₁₂-Alkyl, C₁-C₁₂-Halogenalkyl, C₁-C₁₂-Alkoxycarbonyl und C₁-C₁₂-Alkoxycarbonyl-C₁-C₁₂-alkyl ausgewählt ist;
Ar aus substituiertem oder unsubstituiertem Aryl oder Heteroaryl ausgewählt ist; worin die Substitutionsgruppe, falls vorhanden, aus Halogen, CN, NO₂, C₁-C₆-Alkyl, C₁-C₆-Alkoxy, C₁-C₆-Alkylthio, C₁-C₆-Alkylsulfonyl, C₁-C₆-Alkoxycarbonyl, C₁-C₆-Halogenalkoxy, substituiertem Aryl und substituiertem Aroxyl ausgewählt ist.

2. Verbindung nach Anspruch 1, worin
R₁ aus H, Halogen, NO₂, CN, CONH₂, CH₂CONH₂, CH₂CN, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Alkylcarbonyl, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Halogenalkoxy-C₁-C₆-alkyl und substituiertem oder unsubstituiertem Amino-C₁-C₆-alkyl ausgewählt ist;
R₂ aus H, C₁-C₆-Alkyl, C₁-C₆-Halogenalkyl, C₁-C₆-Alkoxycarbonyl und C₁-C₆-Alkoxycarbonyl-C₁-C₆-alkyl ausgewählt ist;
und worin, wenn Ar aus substituiertem Aryl oder Heteroaryl ausgewählt ist, die Substitutionsgruppe, falls vorhanden, aus Halogen, CN, N02, C₁-C₆-Alkyl, C₁-C₆-Alkoxy, C₁-C₆-Alkylthio, C₁-C₆-Alkylsulfonyl, C₁-C₆-Alkoxycarbonyl, C₁-C₆-Halogenalkoxy, substituiertem Phenyl und substituiertem Phenoxy ausgewählt ist.

3. Verbindung nach Anspruch 2, worin X₁ aus O und NR₂ ausgewählt ist;
R₁ aus H, Halogen, NO₂, CN, CONH₂, CH₂CONH₂, CH₂CN, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Alkylcarbonyl, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Halogenalkoxy-C₁-C₆-alkyl und substituiertem oder unsubstituiertem Amino-C₁-C₃-alkyl ausgewählt ist;
R₂ aus H, C₁-C₆-Alkyl, C₁-C₆-Halogenalkyl, C₁-C₆-Alkoxycarbonyl und C₁-C₆-Alkoxycarbonyl-C₁-C₃-alkyl ausgewählt ist;
und Ar aus substituiertem oder unsubstituiertem Phenyl, Pyridin, Furan, Thiophen und Thiazol ausgewählt ist, worin die Substitutionsgruppe, falls vorhanden, aus Halogen, CN, NO₂, C₁-C₆-Alkyl, C₁-C₆-Alkoxy, C₁-C₆-Alkylthio, C₁-C₆-Alkylsulfonyl, C₁-C₆-Halogenalkoxy, substituiertem Phenyl und substituiertem Phenoxy ausgewählt ist.

4. Verbindung nach Anspruch 3, worin
R₁ aus H, Cl, Br, F, NO₂, CH₂CN und C₁-C₆-Alkyl ausgewählt ist;
R₂ aus H, C₁-C₃-Alkyl, C₁-C₃-Halogenalkyl, C₁-C₃-Alkoxycarbonyl und C₁-C₃-Alkoxycarbonyl-C₁-C₃-alkyl ausgewählt ist;
Ar aus substituiertem oder unsubstituiertem Phenyl, Pyridin, Furan, Thiophen und Thiazol ausgewählt ist, worin die Substitutionsgruppe, falls vorhanden, aus Halogen, CN, NO₂, C₁-C₆-Alkyl, C₁-C₆-Alkoxy, C₁-C₆-Alkylthio, C₁-C₆-Halogenalkoxy, Chlorphenyl und Chlorphenoxy ausgewählt ist.

5. Verbindung nach Anspruch 4, worin
R₁ aus H und Methyl ausgewählt ist;
R₂ aus H, Methyl und Isopropyl ausgewählt ist;
Ar aus substituiertem oder unsubstituiertem Phenyl, Pyridin, Furan und Thiophen ausgewählt ist, worin die Substitutionsgruppe, falls vorhanden, aus Halogen, C₁-C₃-Alkyl, C₁-C₃-Alkoxy, C₁-C₃-Alkylthio und C₁-C₃-Halogenalkoxy ausgewählt ist.

6. Verfahren zur Herstellung einer Verbindung nach einem der Ansprüche 1 bis 5, **dadurch gekennzeichnet, dass** Verbindungen der allgemeinen Formel (I) durch Umsetzung von Azolverbindungen mit einer Hydroxygruppe der allgemeinen Formel (III) mit Halogenmethylbenzol der allgemeinen Formel (IV) in Gegenwart einer Base hergestellt werden: 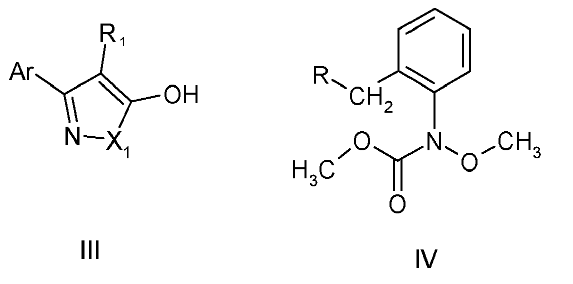 worin
R eine Abgangsgruppe ist.

7. Verfahren nach Anspruch 6, worin R Cl oder Br ist.

8. Verwendung einer Verbindung der allgemeinen Formel (I) nach Anspruch 1 zur Bekämpfung von Insekten bei Pflanzen.

9. Verwendung einer Verbindung der allgemeinen Formel (I) nach Anspruch 1 zur Bekämpfung von Pilzen bei Pflanzen.

10. Fungizid- und Insektizidzusammensetzung, die eine Verbindung der allgemeinen Formel (I) nach Anspruch 1 als Wirkbestandteil umfasst, wobei der gewichtsprozentuelle Anteil des Wirkbestandteils in der Zusammensetzung 0,1 % bis 99 % beträgt.

## Revendications

1. Composé aryl éther **caractérisé en** ayant la formule (I) : 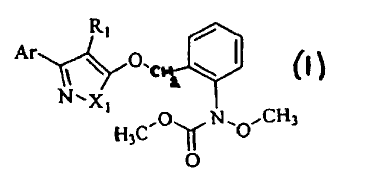 où: X₁ est sélectionné parmi 0, S ou NR₂;
R₁ est sélectionné parmi H, halo, NO₂, CN, CONH₂, CH₂CONH₂, CH₂CN; alkyleC₁-C₁₂, haloalkyleC₁-C₁₂, alkoxyC₁-C₁₂, haloalkoxyC₁-C₁₂, alkylthioC₁-C₁₂, alkylsulfonyleC₁-C₁₂, alkylcarbonyleC₁-C₁₂, alkoxyC₁-C₁₂alkyleC₁-C₁₂, alkoxycarbonyleC₁-C₁₂, alkoxycarbonylC₁-C₁₂alkyleC₁-C₁₂, haloalkoxyC₁-C₁₂alkyleC₁-C₁₂, aminoalkyleC₁-C₁₂ substitué ou non substitué;
R₂ est sélectionné parmi H, alkyleC₁-C₁₂, haloalkyleC₁-C₁₂, alcoxycarbonyleC₁-C₁₂ ou alcoxycarbonyleC₁-C₁₂alkyleC₁-C₁₂; et où
Ar est sélectionné parmi aryle ou hétéroaryle substitué ou non substitué, où le groupe de substitution, si présent, est sélectionné parmi halo, CN, NO₂, alkyleC₁-C₆, alkoxyC₁-C₆, alkylthioC₁-C₆, alkylsulfonyleC₁-C₆, alkoxycarbonyleC₁-C₆, haloalkoxyC₁-C₆, aryle substitué ou aroxyle substitué.

2. Composé selon la revendication 1, où
R₁ est sélectionné parmi H, halo, NO₂, CN, CONH₂, CH₂CONH₂, CH₂CN; alkyleC₁-C₆, haloalkyleC₁-C₆, alkoxyC₁-C₆, haloalkoxyC₁-C₆, alkylthioC₁-C₆, alkylsulfonyleC₁-C₆, alkylcarbonyleC₁-C₆, alkoxyC₁-C₆alkyleC₁-C₆, alkoxycarbonyle C₁-C₆, alcoxycarbonyleC₁-C₆alkyleC₁-C₆, haloalcoxyC₁-C₆alkyleC₁-C₆ ou aminoalkyleC₁-C₆ substitué ou non substitué;
R₂ est sélectionné parmi H, alkyleC₁-C₆, haloalkyleC₁-C₆, alcoxycarbonyleC₁-C₆ ou alcoxycarbonyleC₁-C₆alkyleC₁-C₆; et où si
Ar est sélectionné parmi aryle ou hétéroaryle substitué, où le groupe de substitution, si présent, est sélectionné parmi halo, CN, NO₂, alkyleC₁-C₆, alcoxyC₁-C₆, alkylthioC₁-C₆, alkylsulfonyleC₁-C₆, alcoxycarbonyleC₁-C₆, haloalcoxyC₁-C₆ phényle substitué ou phénoxy substitué.

3. Composé selon la revendication 2, où
X₁ est sélectionné parmi O ou NR₂;
R₁ est sélectionné parmi H, halo, NO₂, CN, CONH₂, CH₂CONH₂, CH₂CN; alkyleC₁-C₆, haloalkyleC₁-C₆, alkoxyC₁-C₆, haloalkoxyC₁-C₆, alkylthioC₁-C₆, alkylsulfonyleC₁-C₆, alkylcarbonyleC₁-C₆, alkoxyC₁-C₆alkyleC₁-C₆, alcoxycarbonyle C₁-C₆, alkoxycarbonyleC₁-C₆alkyleC₁-C_{6,} haloalcoxyC₁-C₆alkyleC₁-C₆ ou aminoalkyleC₁-C₃ substitué ou non substitué;
R₂ est sélectionné parmi H, alkyleC₁-C₆, haloalkyleC₁-C₆, alcoxycarbonyleC₁-C₃ ou alcoxycarbonyleC₁-C₆ alkyleC₁-C₃; et
Ar est sélectionné parmi phényle, pyridine, furanne, thiophène ou thiazole substitué ou non substitué, où le groupe de substitution, si présent, est sélectionné parmi halo, CN, NO₂, alkyleC₁-C₆, alcoxyC₁-C₆, alkylthioC₁-C₆, alkylsulfonyleC₁-C₆, haloalcoxyC₁-C₆, phényle substitué ou phénoxy substitué.

4. Composé selon la revendication 3, où
R₁ est sélectionné parmi H, Cl, Br, F, NO₂, CH₂CN ou alkyleC₁-C₆;
R₂ est sélectionné parmi H, alkyleC₁-C₃; haloalkyle C₁-C₃; alcoxycarbonyleC₁-C₃ ou alcoxycarbonyleC₁-C₃alkyleC₁-C₃;
Ar est sélectionné parmi phényle, pyridine, furanne, thiophène ou thiazole substitué ou non substitué, où le groupe de substitution, si présent, est sélectionné parmi halo, CN, NO₂, alkyleC₁-C₆, alcoxyC₁-C₆, alkylthioC₁-C₆, haloalcoxyC₁-C₆, chloro phényle ou chloro phénoxy.

5. Composé selon la revendication 4, où
R₁ est sélectionné parmi H ou méthyle;
R₂ est sélectionné parmi H, méthyle ou isopropyle;
Ar est sélectionné parmi phényle, pyridine, furanne ou thiophène substitué ou non substitué, où le groupe de substitution, si présent, est sélectionné parmi halo, alkyleC₁-C₃, alcoxyC₁-C₃, alkylthioC₁-C₃ ou haloalcoxyC₁-C₃.

6. Méthode de préparation d'un composé selon l'une quelconque des revendications 1 à 5, **caractérisée en ce que** les composés ayant la formule générale (I) sont préparés par réaction de composés azole contenant un groupe hydroxy ayant la formule générale (III) avec de l'halométhylbenzène ayant la formule générale (IV) en présence de base: 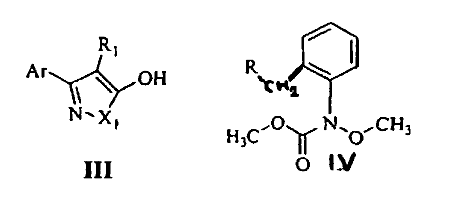 où: R est un groupe mobile.

7. Méthode selon la revendication 6, dans laquelle R est Cl ou Br.

8. Utilisation d'un composé ayant la formule générale (1) selon la revendication 1 pour contrôler des insectes dans des plantes.

9. Utilisation d'un composé ayant la formule générale (I) selon la revendication 1 pour contrôler les champignons dans les plantes.

10. Composition fongicide et insecticide contenant un composé ayant la formule générale (I) tel que défini dans la revendication 1 comme ingrédient actif, où le pourcentage en poids de l'ingrédient actif dans la composition est de 0,1% à 99%.
